(19) Europäisches Patentamt
European Patent Office
Office européen des brevets

(11) Publication number: **0 100 052**
**A1**

(12) # EUROPEAN PATENT APPLICATION

(21) Application number: 83107032.1

(22) Date of filing: 18.07.83

(51) Int. Cl.³: **A 61 K 9/48**, A 61 K 31/557

(30) Priority: 19.07.82 US 399620

(43) Date of publication of application: 08.02.84
Bulletin 84/6

(84) Designated Contracting States: BE CH DE FR GB IT LI LU NL SE

(71) Applicant: SYNTEX (U.S.A.) INC., 3401 Hillview Avenue, Palo Alto, California 94304 (US)

(72) Inventor: Yu, Cheng-Der, 1842 Walnut Drive, Mt. View California 94040 (US)

(74) Representative: Schmied-Kowarzik, Volker, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)

(54) **PGE type compositions encapsulated by acid isolated gelatin.**

(57) Soft-shelled gelatin capsule formulations of PGE-type compounds having superior stability are prepared by using acid isolated gelatin.

EP 0 100 052 A1

BACKGROUND OF THE INVENTION

Field of the Invention

This invention relates to stable soft-shelled gelatin capsule formulations of PGE-type compounds and to a method for preparing stable PGE-type compounds in soft-shelled gelatin capsules. More specifically this invention relates to the use of acid isolated gelatin for the preparation of soft-shelled gelatin, PGE-containing pharmaceutical formulations.

Related Disclosures

Prostaglandins have classically been described as chemically related 20 carbon chain hydroxy fatty acids having the basic skeleton of prostanoic acid:

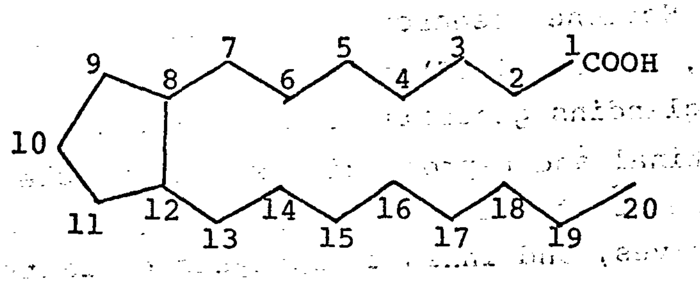

this structure is the basis for prostaglandin numbering and nomenclature.

Naturally occurring prostaglandins are derivatives of prostanoic acid. For descriptive purposes, four types are recognized. The type distinction is based primarily on pentane ring substituents and structural orientation. Although they can be named as derivatives of prostanoic acid, they are conventionally referred to by the letters A, B, E and F. Prostaglandins having a hydroxyl group at the C-11 position and a keto group at the C-9 position are known as PGE or PGE-type compounds. Those having hydroxyl groups at C-9 and C-11 are known as the PGF series and are further designated by an alpha or beta suffix to indicate the configuration of the hydroxyl group at said position. Series A and B have a keto group at C-9 and a double bond between C-10 and C-11 or C-8 and C-12 respectively. The natural compounds are the alpha-hydroxy substituted compounds. Prostaglandins may contain different series of unsaturation in the molecule, particularly at C-5, C-13 and C-17. The unsaturation is also indicated by a suffix. Thus, for example, $PGF_1$ and $PGE_1$ series refer to prostanoic acids having a trans-olefin bond at the C-13 position, while the $PGF_2$ and $PGE_2$ series refer to prostadienoic acids having a cis-olefin bond at the C-5 position and a trans-olefin bond at the C-13 position. For a review on prostaglandins and the definition of primary prostaglandins, see for example, S. Bergstrom, Recent Progress in Hormone Research 22, pp. 153-175 (1966) and Science 157, p. 382 (1967) by the same author.

Prostaglandins generally act to stimulate gastrointestinal and reproductive smooth muscles, affect relaxation and contraction of respiratory smooth muscle, are hypotensives, and inhibit lipolysis of fatty acids, gastric acid secretion and blood platelet aggregation. There is not a precise structure-activity relationship in

the prostaglandin family as much cross-activity is evident.

A great number of studies have been undertaken to enhance, extend and otherwise modify the activity of naturally occurring prostanoic acids. The majority of these studies have focused on modification of two areas, the two side chains and substituents attached to the cyclopropane moiety [see, for example, U. Axen et al, Synthesis Vol. 1, John Wiley and Sons Inc., New York, NY 1973 and P. H. Bently, Chem. Soc. Reviews, 2, 29 (1973)].

Of special interest to this invention is that group of prostaglandins which are labile in most standard pharmaceutical compositions, particularly PGE compounds and PGE-type compounds. In many instances the cyclopentane ring substituents substantially affect the prostaglandin's level of activity. Compounds which lose an oxygen from either C-9 or C-11 on the cyclopentane ring or which have these positions altered show altered levels of activity. For instance $PGE_2$alpha, which has a carbonyl group at C-9 and a hydroxyl group at C-11 stimulates smooth muscle tissue but loss of the C-11 hydroxyl group to give a double bond in the cyclopentane ring, the PGA or PGB forms, show little or no such activity. This conversion is chemically facile because of the presence of the carbonyl group at C-9 in the PGE and PGE-type compounds which makes the hydroxyl group at C-11 labile to either base or acid dehydroxylation. The product of this dehydroxylation is a double bond conjugated with the carbonyl group of C-9, a stable chemical entity. Under acid conditions PGE-type compounds convert readily to the PGA form. Basic conditions cause PGE-type compounds to dehydroxylate and rearrange to the PGB form. In the case of $PGE_2$ type compounds this latter form is particularly stable because the C-9 carbonyl is now conjugated with the C-8/C-12 and

C-13/C-14 double bonds. Similar degradation patterns have been observed in most compounds which have PGE-type cyclopentane ring substituents.

Initial efforts at providing easily dispensable dose formulations of prostaglandins, particularly for PGE-type compounds, met with difficulty. Aqueous PGE solutions were found to undergo rapid loss of activity when stored at temperatures above 0°C at any pH, but particularly under alkaline conditions. Hydrous solutions adjusted to pH 5-7 were found to be most stable but loss of activity was still so rapid, drug concentrations after several months were very uncertain. Even in neutral or neat solutions there was found gradual degradation. Similar rapid degradation under these conditions have been observed in most compounds which have PGE-type cyclopentane ring substituents.

Various attempts have been made to formulate stable solutions of PGE-type compounds. Stabilization of these compounds has been observed in some solutions and in solid form at -20°C or lower. More practical and usable alternative methods for stabilizing these prostaglandins have been developed and are described, for example, in U.S. Pat. Nos. 3,749,800; 3,826,823; 3,829,579; 3,851,052; 3,833,725; and 4,221,793. These patents teach the use of such solvents as lower molecular weight alcohols, polyalkylene glycols, dialkylated polyalkylene glycols, triacetin, dimethylacetamide and triethylcitrate.

Prostaglandins are not always readily susceptible of powder and pill formulation because their potency creates cross-contamination problems which are more easily addressed by solution formulation. While these solutions may be provided in stock form in a bottle or other container, it is more convenient for the user and pharmaceutically more elegant to provide consumer with a prepared unit dose in some form rather than to have to

make individual measurements each time he or she takes the medicine. The most convenient unit dose form for solution is the soft-shelled gelatin capsule. While this formulation type has been suggested and described previously, see U.S. patent 3,966,962 and Yalkowsky and Roseman (J. Pharm. Sci. 68, 114-115, 1979) for example, it has now been found that all gelatin is not equal.

Basically there are two types of gelatin, denoted Type A and Type B, which designations are predicated on the fact in one instance the gelatin is processed from raw materials with acid, Type A and the other type, Type B, is processed with base, usually lime. Comparative stability studies of PGE compound containing soft-shelled gelatin capsules demonstrate that Type A gelatin-based capsules have unexpected and significantly superior active ingredient stability versus Type B gelatin-based capsules. Therefore it is the purpose of this invention to disclose formulations and methods for preparing stable PGE-type compound formulations of soft-shelled gelatin capsules utilizing Type A, acid treatment prepared, gelatin.

## SUMMARY

One aspect of this invention relates to an improved PGE-type compound-containing soft-shelled gelatin capsule formulation comprising a PGE-type compound, a PGE stabilizing, soft-shelled-gelatin-compatible solvent and soft-shelled gelatin capsule forming material wherein the improvement comprises using acid treatment prepared gelatin in the capsule forming material.

In a second aspect this invention relates to a method for stabilizing PGE-type compositions contained in a soft-shelled gelatin capsule which method comprises encapsulating a soft-shelled gelatin capsule compatible, PGE compound stabilizing solvent containing said compound

with a soft-shelled gelatin capsule forming material wherein the gelatin is acid treatment prepared gelatin.

DESCRIPTION OF THE INVENTION

The novel aspect of this invention is the use of acid treatment prepared gelatin for making soft-shelled gelatin capsules of PGE or PGE-type compounds. It has been found that acid treatment prepared gelatin, or Type A gelatin, unexpectedly provides a capsule formulation having a stability profile equal to the drug/solvent mixture alone, while the same formulation, but prepared with Type B gelatin showed greatly accelerated active ingredient degradation.

The practice of this invention must also take into account the compatibility of the gelatin and gelatin capsule formulating compositions with the solvent to be encapsulated as not all PGE stabilizing solvents are necessarily compatible with soft-shelled gelatin capsule forming materials.

Gelatin is a generic term used to describe a product obtained by partial hydrolysis of collagen derived from skin white connective tissue and bones of animals. This product is comprised of a mixture of peptide fragments varying in molecular weight between about 15,000 and 250,000 which can form a reversible hydrogel with water or be polymerized with polyhydric additives to form an irreversible gel. It is this latter form, particularly, which has utility for preparing pharmaceutical formulation. Gelatin designated Type A and Type B are the two commercially available gelatins for preparing soft-shelled gelatin capsules. Type A gelatin usually is made from the skins and bones of animals, primarily pigs, by a process which calls for first digesting stock material in dilute mineral acid for an appropriate period, hence the designation, Type A gelatin or, alternatively, acid treatment prepared gelatin. An

alternative method for preparing gelatin is to first process the raw material with calcium hydroxide and is thus designated Type B gelatin. As a result of the manufacturing differences, Type A gelatin has a relatively low pH and a relative high isoelectric point while Type B exhibits the opposite characteristics. Nevertheless, their amino acid compositions are about the same.

However, it has now been found that soft-shelled gelatin encapsulated PGE compounds have a different stability depending on the type of gelatin used. Unexpectedly it has been found that Type A gelatin encapsulated PGE-type prostaglandin compositions essentially retain the stabilizing effects of the solvent in which they are dissolved, as opposed to Type B gelatin based soft-shelled capsules which show an accelerated degradation rate. Study results can be found in the Examples set out hereinafter.

Type A gelatin is commercially available from a large number of manufacturers whose addresses are available through the Gelatin Manufactures Institute of America, Inc. A representative number of firms are for example: Atlantic Gelatin, General Foods Corp., Hillstreet, Woburn, MA 01801; Keystone Co., 2350 Kerper Blvd., Dubuque, IA 52001; Kind and Knox Gelatin Company, 900 Kings Highway, Cherryhill, NJ 08034, all representative of a few of the 14 or more companies which produce edible gelatin. The address for the Gelatin Manufacturers Institute of America is 516 Fifth Avenue, Room 507, New York, NY 10036. Any or all of these commercial organizations can provide Type A gelatin.

The process for preparing Type A gelatin is, generally, to wash animals skins, bone and other collagen material in cold water for a few hours to remove extraneous matter. The stock is then digested in dilute

mineral acid (HCl, $H_2SO_4$, $H_2SO_3$, $H_2PO_4$, pH 1-3) at about room temperature until maximum swelling has occurred. This requires a period of approximately 24 hours. The swollen stock is then washed with water to remove excess acid and adjusted to a pH of 3.5-4.0 for the recovery of gelatin by hot water extraction. Hydrolytic extraction is carried out in batch-type operations with successive portions of hot water at progressively higher temperatures until the maximum yield is obtained. Gelatin solution is then chilled into gelled sheets, dried and then ground. Typical properties of Type A gelatin are listed in Table I.

TABLE I

Typical Properties of Type A Gelatin

| Property | Gelatin, USP Type A |
|---|---|
| Residue on Ignition | 0.23% |
| Sulfur Dioxide | 0.0010% |
| Arsenic | 0.8 PPM |
| Heavy Metals | 15 - 20 PPM |
| PH (6.7% Soln.) | 3.8 - 6.0 |
| Loss on Drying | 11.6% |
| Iron | 9 PPM |
| Oxidizing Substance | None |
| Reducing Substance | 0.0010% |
| Isoelectric Point | 7.0 - 9.2 |

Soft, or elastic, shelled gelatin capsule forming material is comprised of three basic components: gelatin; a plasticizer; and water but may contain additional or optional ingredients such as preservatives, coloring agents, flavorings, sugars or the like. However for the purpose of this invention it is preferable to keep the number and type of ingredients making up the soft-shelled

gelatin capsule forming material as few and simple as possible to guard against introduction of any material which may have an adverse effect on PGE stability.

The development of a gelatin capsule appropriate for the type of solvent being used can best be determined at the time and under the circumstances needed to prepare the gelatin capsules according to the practice of this invention. Any plasticizer normally useful in preparing soft-shelled gelatin capsules such as, for example, glycerin, sorbitol and pharmaceutical grade sorbitol special in combinations thereof may be used for the practice of this invention. This list is intended to be representative of plasticizers which may be used and is not exhaustive of such compounds or limitative thereof. The choice of plasticizer will be related to the type and nature of the encapsulated material while the relative ratio of plasticizer to gelatin will determine the hardness of the capsule. Both items are best determined as needed to encapsulate a particular solvent/PGE preparation.

The use of preservatives or other additives such as coloring agents, flavoring agents or the like, are optional with the practitioner of this invention. Certain of the studies disclosed herein indicate parabens may have some small effect on PGE stability but their use is not mandatory in the practice of this invention. Additives are optional with the practitioner though there is no guarantee that the stabilizing effects seen with Type A gelatin will necessarily be retained or enhanced with each and every additive.

A soft-shelled gelatin capsule forming mass, as prepared prior to capsule formulation, will comprise between about 24-50% by dry weight gelatin, about 15-44% plasticizer by dry weight and about 32-35% by weight water. Optional components, e.g. preservatives or other

additives, may be added in an amount of about 0.01 to 0.5% by weight, an appropriate modification being made in the percentage of other components as necessary to realize 100%. These figures represent percentages which should give satisfactory capsules, after drying, for all solvent compositions within the scope of this invention, though additional gelatin capsule forming masses which do not fall precisely within these figures may be necessary or indicated for a particular solvent. Such masses, dictated by solvent/plasticizer choices, "hardness" consideration or the like, should be considered within the scope of this invention because it is the use of Type A gelatin which is of importance, not its precise percentage in the encapsulating mass.

Formation and filling of soft-shelled gelatin capsules is a well-known art originally involving manual preparation but now expanded to a number of different mechanical processes. For example, the oldest illustrated process is the so-called plate process which is still in use though subsequent processes such as the rotary die process, reciprocating die process, and the Accogel machine processes have carried the art of capsule formation into the age of automation. Any of these processes or any other formulation method may be employed in the practice of this invention.

Newly prepared capsules may be washed, dried and otherwise treated in accordance with standard practices in this art to make them ready for bottling and storage. The compositions of this invention do not require any special handling procedures, materials, drying temperature or the like.

A soft-shelled gelatin compatible, PGE stabilizing solvent may be any pharmaceutically acceptable non-toxic solvent which in and of itself provides stable PGE and PGE-type compound compositions, but is additionally

compatible with soft-shelled gelatin capsule compositions. A number of solvents have been investigated and reported on as being appropriate for preparing stable PGE solutions or suspensions and all may be used in the practice of this invention provided they are additionally compatible with the encapsulating material. For example, there may be used such solvents as triacetin, ethylene glycol, propylene glycol, polyalkylene glycols, dialkylated glycols, the triesters of citric acid, or alkylcarbonate diesters, either the cyclic or linear form. Ethanol and other lower alcohols have been reported as PGE stabilizing solvents but all may not be compatible with soft-shelled gelatin capsule formulations. Particularly preferred is propylene carbonate. The solvents may be used neat, with additives or in combinations or mixtures. Additionally they may have water present, provided such water does not affect composition stability to an unacceptable degree.

The soft-shelled gelatin capsule formulations of this invention may be used for all types of prostaglandin compositions but has the greatest utility for PGE compounds and PGE-type compounds. The phrase "PGE compounds" refers to those naturally occurring compounds which are derivatives of prostanoic acid and which have a C-9 carbonyl substituent and C-11 and C-15 hydroxyl substituents. These compounds have varying degrees of unsaturation as discussed above and all are intended to be included within the scope of the phrase "PGE compounds". There is intended to be included in this definition $PGE_1$, $PGE_2$, $PGE_3$ and dihydro-$PGE_1$ compounds. Esters of these compounds have been synthetically prepared, see for example U.S. Pat. Nos. 3,069,332 and 3,598,858.

There also have been prepared many compounds which retain the C-9 carbonyl and C-11 hydroxyl cyclopentane

ring structural features but wherein the side chains have been modified; and which cause at least part of the biological response caused by PGE compounds. These compounds are intended to be included within the scope of this invention and are covered herein by the phrase "PGE-type compounds". Modified compounds differ from PGE compounds in one or more structural aspects, for example, in having one or more substituents, for example, alkyl, fluoro, phenyl, or cycloalkyl, on one or both side chains; in having fewer or more methylene groups in one or both side chains; in having a hetero atom, for example, oxygen in place of a side-chain methylene group; in having cis rather than a trans or a trans rather than a cis configuration for a side-chain carbon-carbon double bond; in having allenic double bonds in one side chain; or in any combination of those structural aspects. As examples of art which discloses such PGE-type compounds and others, see U.S. Pat. Nos. 3,639,463; 3,759,978; 3,767,695; 3,781,325; 3,804,889; 3,812,179; 3,813,433; 3,833,640; 3.835,180; 3,842,118; 3,847,966; 3,849,487; 3,855,270; 3,864,387; and 4,178,457. See also German Offenlegungschrift Nos 1,937,675; 1,937,921; 2,011,969; 2,036,471; 2,118,686; 2,121,980; 2,144,048; 2,150,361; 2,154,309; 2,165,184; 2,209,990; 2,217,044; 2,221,443; 2,317,019; 2,320,552; 2,322,673; 2,332,400; 2,345,685; 2,423,155 and 2,423,156. See also French Pat. No. 2,119,855, Belgian Pat. Nos. 779,898 and 782,822.

Also, for the purposes of this invention, it is intended to include racemic mixtures as well as resolved enantiomers of both PGE and PGE-type compounds.

In both instances it should be understood that not only the carboxylic acids are to be included but also esters of said compounds. Those esters wherein the esterifying radical is alkyl of 1 to 12 carbon atoms, inclusive, cycloalkyl of 3 to 10 carbon atoms, aralkyl of

7 to 12 carbon atoms, phenyl, and phenyl substituted with 1, 2 or 3 chloro or alkyl of 1 to 4 carbon atoms are typical. Alkyl esters of 1 to 4 carbon atoms are particularly useful, especially methyl and ethyl esters.

Pharmaceutically acceptable salts of both compound groups are also to be included. These salts may be prepared from pharmaceutically acceptable non-toxic bases, including inorganic and organic bases. Salts derived from inorganic bases include, preferably, ammonium, potassium, sodium, calcium and magnesium salts. Preferred organic bases are isopropylamine, diethylamine, ethanolamine, peperidine, tromethamine, choline and caffeine.

Of particular interest are stable compositions of PGE-type compounds wherein the prostaglandins are 16-phenoxy and 16-substituted phenoxy prostaglandin E analogs represented by the following formula:

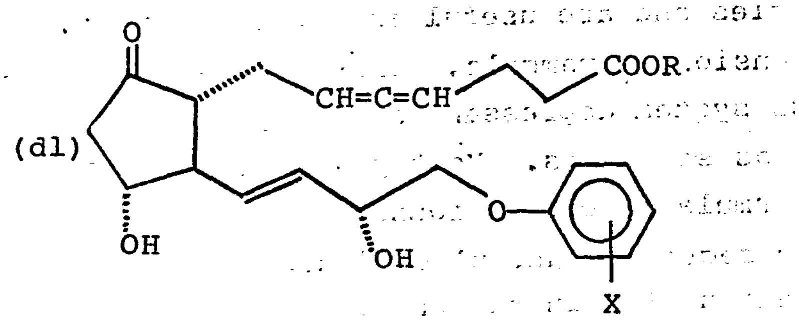

I

wherein R is hydrogen, a lower alkyl group of 1 to 4 carbon atoms, or the pharmaceutically acceptable, non-toxic salts of compounds in which R is hydrogen; and X is hydrogen, o-, m- or p-halo (fluoro, chloro or bromo), o, m- or p-methyl or o-, m- or p-methoxy.

The lines shown in the above formula and in the formulas below as "≡" indicate that the substituents are in alpha configuration, i.e., below the plane of the cyclopentane ring.

The double bond at C-13 has the same configuration as in natural prostaglandins of the PGE and PGF series, that is the trans configuration.

These novel compounds possess asymmetric centers and thus can be produced as racemic "(dl)" mixtures or as individual 8R-antimers. The racemic mixtures can be resolved if desired at appropriate stages by methods known to those skilled in the art, to obtain the respective individual antimers.

These particular compounds exhibit prostaglandin-like biological activity and thus are useful in the treatment of mammals where the use of prostaglandins is indicated. These compounds are useful for the control of asthmatic attack because they are bronchodilators and they also exhibit antiallergic properties by inhibition of mediator release. In addition, they are also useful for treating mammals for bronchial spasm or wherever bronchodilator compounds also exhibit vasodilator properties and are useful in controlling palpitating hypertension in mammals. they further exhibit central nervous system depressant activity in mammals, and are useful as sedatives. Most particularly, compounds of this formula have been found to be potent inhibitors of gastric secretion and ulcer induction and thus are extremely useful in the treatment and prevention of gastric and duodenal ulcers. The compounds are the subject of U.S. Patent No. 4,178,457 which is incorporated herein by reference.

In the practice of this invention, prostaglandin concentrations may range from 0.001 to 100 mg/ml of chosen solvent. While the particular concentration for a given prostaglandin will depend on its inherent level of activity and the therapeutic dose to be administered at a particular time, a preferred concentration range will be between 0.01 mg/ml and 20 mg/ml. The most preferred

concentration range is about 0.01 mg/ml to 5.0 mg/ml, particularly for the compounds represented by Formula I.

SPECIFIC EMBODIMENTS OF THE INVENTION

In the general case, the compositions of this invention are prepared by adding the prostaglandin to the solvent at the desired concentration and stirring the admixture at room temperature until a homogeneous solution is obtained. This solution is then filled into soft-shelled gelatin capsule forming material by any of the various methods of manufacturing soft-shelled gelatin capsules including the rotary die process, the Accogel machine or by any machine or hand preparation methods.

The following examples set out specific descriptions of means for practicing the invention described herein and result obtained using these compositions and methods.

EXAMPLE I

To 10 ml of anhydrous propylene carbonate at 25°C was added from about 0.01 to 10 mg of (dl)-9-keto-11,15-dihydroxy-16-phenoxy-17,18,19,20-tetranorprosta-4,5,13-trans-trienoic acid methyl ester. The mixture was stirred with a blade type stirrer for about 15 minutes until an homogeneous solution is obtained. This mixture was then filled into soft-shelled gelatin capsules comprised of Type A, or Type B gelatin, (45.5% weight/weight (w/w)), glycerin (11.3% w/w), sorbitol (11.3% w/w), methylparaben (0.16% w/w), propylparaben (0.04% w/w) and water 31.8% (w/w). These capsules were stored at 5°C, 22°C temperature, 30°C and 40°C for up to 12 months during which time assays were carried out on aliquots. Data for the various time points, temperatures and compositions are given in Table I below.

TABLE I

Stability of an E-type Prostaglandin[a] in Propylene Carbonate (0.25 mg/ml) Encapsulated in Soft Elastic Type A Gelatin and Type B Gelatin Capsules (with Parabens)

| | % Theoretical Conc. | | | | | | | |
|---|---|---|---|---|---|---|---|---|
| | 40°C | | 30°C | | 22°C | | 5°C | |
| Months | A | B | A | B | A | B | A | B |
| 1.0 | 103.0 | - | - | - | - | - | - | - |
| 2.0 | 104.0 | - | 105.0 | - | 106.0 | - | - | - |
| 3.0 | 98.0 | - | - | - | - | - | - | - |
| 3.5 | - | 73.5 | - | 86.5 | - | - | - | - |
| 4.0 | 97.0 | - | 103.0 | - | 107.0 | 92.0 | - | 104.5 |
| 5.0 | 90.5 | 58.0 | - | - | - | - | 105.0 | - |
| 6.0 | 85.0 | - | 99.0 | - | 102.5 | - | - | - |
| 7.0 | - | 40.5 | - | 76.0 | - | 91.0 | 102.5 | - |
| 9.0 | 75.0 | 24.5 | 97.0 | 70.0 | 100.0 | 91.0 | - | - |
| 9.5 | - | - | - | - | - | - | 101.0 | - |
| 10.0 | - | - | - | - | - | - | - | 101.5 |
| 12.0 | - | 11.5 | - | 65.0 | - | - | - | - |

[a] (d,l)-9-keto-11,15-dihydroxy-16-phenoxy-17,18,19,20-tetranorprosta-4,5,13-trans-trienoic acid methyl ester.

EXAMPLE II

Soft-shelled gelatin capsules containing 0.25 mg/ml of the trienoic acid ester recited in Example I in propylene carbonate were prepared as per Example I but without preservatives (parabens) or other additives. Seven month stability results at several temperatures are given in Table II below.

TABLE II

Stability of an E-type Prostaglandin[a] in Propylene Carbonate (0.25 mg/ml) Encapsulated in Soft Elastic Type A Gelatin and Type B Gelatin Capsules (Preservative-Free)

| | % Theoretical Conc. | | | | | | | | | |
|---|---|---|---|---|---|---|---|---|---|---|
| | 50°C | | 40°C | | 30°C | | 22°C | | 5°C | |
| Mons | A | B | A | B | A | B | A | B | A | B |
| 1 | 104.0 | 93.0 | 102.0 | 99.0 | - | - | - | - | - | - |
| 2 | 93.0 | 67.0 | 102.0 | 94.0 | 103.0 | 100.0 | 104.0 | 103.0 | - | - |
| 3 | 85.5 | 46.5 | 99.5 | 87.5 | - | - | - | - | 105.3 | 104.7 |
| 4 | - | - | 98.0 | 81.0 | 100.5 | 95.0 | 105.5 | 102.0 | - | - |
| 5 | - | - | 96.0 | 75.0 | - | - | - | - | 106.0 | 106.5 |
| 6 | - | - | 93.5 | 69.0 | 99.0 | 91.0 | 104.5 | 99.5 | - | - |
| 7 | - | - | - | - | - | - | - | - | 105.0 | 104.0 |

[a] (d,l)-g-keto-11 ,15 -dihydroxy-16-phenoxy-17,18,19,20-tetranorprosta-4,5,13-trans-trienoic acid methyl ester.

EXAMPLE III

A solution of (dl)-9-keto-11 ,15 -dihydroxy-16-phenoxy-17,18,19,20-tetranorprosta-4,5,13-trans-trienoic acid methyl ester was prepared as a solution in propylene carbonate at a concentration of 0.5 mg/ml. This solution was divided amongst a number of glass vials and stored at various temperatures. The temperature and % active ingredient remaining are given in Table III.

TABLE III

Stability of an E-type Prostaglandin[a] in Propylene Carbonate (0.5 mg/ml)

| Time (Months) | % Remaining of Initial Drug Conc. | | | | |
|---|---|---|---|---|---|
| | At 80°C | At 60°C | At 50°C | At 40°C | At 22°C |
| 0 | 100 | 100 | 100 | 100 | 100 |
| 0.25 | 98.3 | -- | -- | -- | -- |
| 0.5 | 97.6 | 100.2 | -- | -- | -- |
| 0.75 | 97.4 | -- | 99.3 | -- | -- |
| 1 | 96.2 | 96.7 | -- | -- | -- |
| 2 | 92.5 | 94.8 | 97.9 | 99.6 | -- |
| 3 | 90.4 | 92.8 | -- | -- | -- |
| 4 | -- | 87.3 | 97.1 | 101.3 | -- |
| 5 | -- | 86.3 | -- | -- | -- |
| 6 | -- | -- | 95.9 | 96.8 | 98.9 |
| 12 | -- | -- | -- | 98.3 | 99.0 |

a (d,l)-9-keto-11 ,15 -dihydroxy-16-hydroxy-17,18,19,20-tetranorprosta-4,5,13-trans-trienoic acid methyl esters.

WHAT IS CLAIMED IS:

1. An improved PGE-type compound containing soft-shelled gelatin capsule formulation, comprising a PGE-type compound, a PGE stabilizing, soft-shelled gelatin compatible solvent and soft-shelled gelatin capsule forming material, wherein the improvement comprises using acid treatment prepared gelatin in the capsule forming material.

2. The composition of Claim 1 wherein said solvent is triacetin, a carbonate diester, a polyalkylene glycol, a dialkylated polyalkylene glycol or trialkyl citrate, preferably propylene carbonate.

3. The composition of Claim 1 or 2 wherein said PGE compound is a compound according to the formula

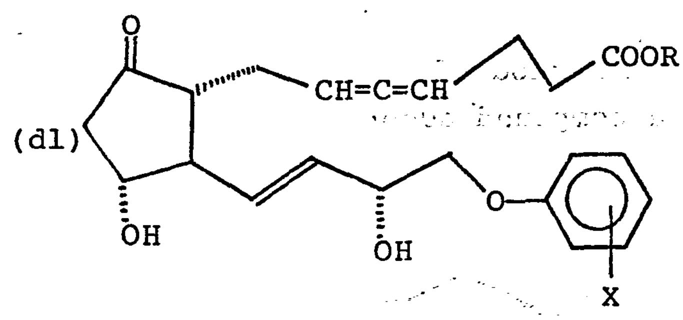

wherein R is hydrogen, a lower alkyl group of 1 to 4 carbon atoms, or the pharmaceutically acceptable, non-toxic salts of compounds in which R is hydrogen; and X is hydrogen, o-, m- or p-halo (fluoro, chloro or bromo), o, m- or p-methyl or o-, m- or p-methoxy.

4. The composition of Claim 1 or 2 wherein said PGE compound is (dl)-9-keto-11 ,15 -dihydroxy-16-phenoxy-17,18,19,20-tetranorprosta-4,5,13-trans-trienoic acid methylester in a concentration of between 0.001 mg/ml to 100 mg/ml, preferably between 0.01 mg/ml to 20 mg/ml, most preferably between 0.01 mg/ml to 5.0 mg/ml.

5. A method for stabilizing PGE type compound containing soft-shelled gelatin capsules, which method comprises encapsulating a soft-shelled gelatin compatible PGE-type compound stabilizing solvent containing said PGE-type compound with soft-shelled gelatin capsule forming material wherein the gelatin is acid treatment prepared gelatin.

6. The method of Claim 5 wherein said solvent is triacetin, a carbonate diester a polyalkylene glycol, a dialkylated polyalkylene glycol or trialkyl citrate, preferably propylene carbonate.

7. The method of Claim 5 or 6 wherein said PGE compound is a compound according to the formula

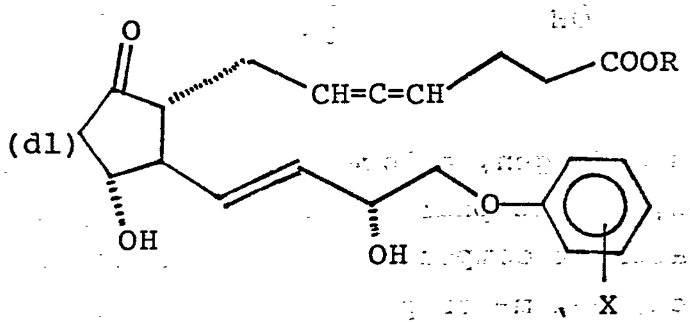

wherein R is hydrogen, a lower alkyl group of 1 to 4 carbon atoms, or the pharmaceutically acceptable, non-toxic salts of compounds in which R is hydrogen; and

X is hydrogen, o-, m- or p-halo (fluoro, chloro or bromo), o, m- or p-methyl or o-, m- or p-methoxy.

8. The method of Claim 5 or 6 wherein said PGE compound is (dl)-9-keto-11 ,15 -dihydroxy-16-phenoxy-17,18,19,20-tetranorprosta-4,5,13-trans-trienoic acid methylester in a concentration of between 0.001 mg/ml to 100 mg/ml, preferably between 0.01 mg/ml to 20 mg/ml, most preferably between 0.01 mg/ml to 5.0 mg/ml.

European Patent Office

# EUROPEAN SEARCH REPORT

0100052

Application number

EP 83 10 7032

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X<br>Y | US-A-3 966 962 (S.H. YALKOWSKY)<br>* Column 2, lines 51-58; column 9, lines 44-56; claims 1-5 *<br>--- | 1-2,4-6 | A 61 K 9/48<br>A 61 K 31/557 |
| D,X<br>Y | JOURNAL OF PHARMACEUTICAL SCIENCES, vol. 68, no. 1, January 1979, pages 114-115, Washington, D.C., US<br>S.H. YALKOWSKY et al.: "Stability of E-type prostaglandins in triacetin" * Whole article *<br>--- | 1-2,4-6 | |
| D,X<br>Y | US-A-4 211 793 (S.A. LODHI)<br>* Column 3, lines 31-39; example 4; claims *<br>--- | 1-2,4-6 | TECHNICAL FIELDS SEARCHED (Int. Cl. 3) |
| X,Y | US-A-4 328 245 (C.D. YU)<br>* Claims *<br>--- | 1-8 | A 61 K 9/00<br>A 61 K 31/00 |
| Y | DR. HERBERT P. FIEDLER: "Lexikon der Hilfstoffe für Pharmazie, Kosmetik und angrenzende Gebiete", vol. 9, 1971, pages 234-235, Editio Cantor KG, Aulendorf, DE<br>* Page 234: Gelatine *<br>--- -/- | 1,5 | |

The present search report has been drawn up for all claims

| Place of search | Date of completion of the search | Examiner |
|---|---|---|
| THE HAGUE | 07-10-1983 | BERTE M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0100052

Application number

EP 83 10 7032

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | US-A-3 456 051 (H.M. HYOGO) * Column 1, lines 23-30; column 1, line 64 - column 2, line 68; column 3, lines 31-38; claim 1 * --- | 1,5 | |
| Y | CHEMICAL ABSTRACTS, vol. 94, no. 10, March 9, 1981, page 384, no. 71529f, Columbus, Ohio, US & JP - A - 80 138 457 (TOKAI CAPSULE CO., LTD.) 29-10-1980 * Whole abstract * ----- | 1,5 | |
| | | | TECHNICAL FIELDS SEARCHED (Int. Cl. 3) |

The present search report has been drawn up for all claims

| Place of search | Date of completion of the search | Examiner |
|---|---|---|
| THE HAGUE | 07-10-1983 | BERTE M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82